# FASCICULE DE BREVET EUROPEEN

(11) **EP 3 390 326 B1**
(45) Date de publication et mention de la délivrance du brevet: **20.11.2019**
(21) Numéro de dépôt: 16809088.4
(22) Date de dépôt: 13.12.2016
(51) Int. Cl.: C07C 1/213

(54) **PROCEDE DE PRODUCTION DE BUTADIENE A PARTIR DE BUTANEDIOLS**
VERFAHREN ZUR HERSTELLUNG VON BUTADIEN AUS BUTANDIOLEN
METHOD FOR PRODUCING BUTADIENE FROM BUTANEDIOLS

(30) Priorité: 18.12.2015 FR 1562859
(43) Date de publication de la demande: 24.10.2018
(73) Titulaire: IFP Energies nouvelles, 92500 Rueil-Malmaison (FR); Compagnie Générale des Etablissements Michelin, 63000 Clermont-Ferrand (FR)
(72) Inventeur: PACHECO, Nuno, 63040 Clermont-ferrand Cedex 9 (FR); DORATO, Margarita, 63040 Clermont-ferrand Cedex 9 (FR); JACQUIN, Marc, 69002 Lyon (FR); DASTILLUNG, Rejane, 69005 Lyon (FR); COUDERC, Sophie, 92200 Neuilly Sur Seine (FR)
(74) Mandataire: IFP Energies nouvelles
(86) Numéro de dépôt international: PCT/EP2016/080824
(87) Numéro de publication internationale: WO 2017/102743

(56) Documents cités:
- US-A- 2 383 205

## Description

### DOMAINE DE L'INVENTION

L'invention concerne le procédé de production de 1,3-butadiène à partir d'une charge butanediol comprenant au moins un butanediol choisi dans la liste constituée par le 1,4-butanediol, le 2,3-butanediol, de 1,3-butanediol et leurs mélanges.

### ART ANTÉRIEUR

Aujourd'hui, 95% de la production de 1,3-butadiène est assurée par le vapocraquage d'hydrocarbures et l'extraction subséquente des dioléfines au sein d'une coupe de distillation C₄ par des procédés de distillation extractive.

L'évolution du prix des matières premières a conduit à opérer les unités de vapocraquage avec des charges de plus en plus légères, car moins coûteuses, entrainant la diminution de production de coupe C₄, et par conséquent de 1,3-butadiène.

D'autres procédés permettent de produire du butadiène à l'échelle industrielle. On peut citer les procédés de déshydrogénation des butènes et des butanes, à partir d'une ressource hydrocarbures en C₄. On peut aussi mentionner le procédé Lebedev, qui permet d'obtenir du 1,3-butadiène à partir d'éthanol.

Un autre procédé de production de 1,3-butadiène a été opéré à l'échelle pilote dans les années 1945 aux USA, et décrit par exemple dans les brevets FR 859902, US 2383205, US 2372221, et dans Industrial & Engineering Chemistry, 37 (9), 1945, p.865 à 908. Ce procédé est constitué de 2 étapes principales :
- l'estérification du 2,3-butanediol par un acide carboxylique pour former le diester correspondant
- la pyrolyse du diester pour produire du 1,3-butadiène et de l'acide carboxylique qui est recyclé à l'étape d'estérification.

Ce procédé a été mis au point car la déshydratation directe du 2,3-butanediol conduit à la formation très majoritaire de methyl-ethyl-cétone (MEK), et que la MEK ne peut pas être déshydratée en 1,3-butadiène. Ce procédé est particulièrement intéressant car l'étape de pyrolyse du diester peut être réalisée avec de très bons rendements (typiquement plus de 80%), et le 1,3-butadiène obtenu est de grande pureté (typiquement plus de 99%), ce qui est crucial pour son utilisation dans diverses applications (chimie fine, élastomère).

Une difficulté soulevée par le procédé de production de 1,3-butadiène à partir d'un des isomères du butanediol (1,4-butanediol, 2,3-butanediol et 1,3-butanediol) par estérification puis pyrolyse est la purification de l'acide carboxylique en vue de son recyclage. La distillation azéotropique hétérogène mise en œuvre pour sécher l'acide carboxylique, c'est à dire éliminer l'eau produite à l'étape d'estérification, est difficile à opérer, notamment du fait de l'accumulation de composés organiques dans l'acide carboxylique.

En effet, ces composés organiques, en s'accumulant dans l'acide carboxylique, perturbent les équilibres liquide-liquide du système eau / acide carboxylique / entraineur, voire homogénéisent un système qui est diphasique en leur absence, et donc rendent le séchage de l'acide carboxylique par distillation azéotropique hétérogène inopérant.

Les composés organiques en question peuvent être :
1. des sous-produits de déshydratation des butanediols (THF pour une charge 1,4-butanediol, MEK pour une charge 2,3-butanediol, et 1-buten-4-ol pour une charge 1,3-butanediol) formés notamment dans l'étape d'estérification ;
2. des intermédiaires de pyrolyse et des sous-produits formés à l'étape de pyrolyse (comme par exemple le vinylcyclohexène, le méthylvinylcarbinol acétate (MVCA), le méthyléthylcétoneénol acétate (MEKEA), le crotylacétate (CA), le VCH, la MEK ou la méthylacétylacétone (MAA)), qui sont difficilement séparables de l'acide carboxylique libéré à l'étape de pyrolyse, et qui peuvent donc être renvoyés, totalement ou partiellement, à l'étape d'estérification.

Ces nombreux intermédiaires de pyrolyse ont une température d'ébullition proche de celle de l'acide acétique, et forment des azéotropes homogènes (maximum et minimum) avec ce dernier. La purification de l'acide acétique ne peut donc se faire par simple distillation. Le brevet US 2,372,221 décrit l'utilisation du liquide pyrolytique sans purification préalable directement à l'étape d'estérification, et les conséquences catastrophiques sur la distillation azéotropique hétérogène mise en œuvre (entraineur = benzène dans ce cas) pour sécher l'acide carboxylique.

Dans le procédé de l'art antérieur (Industrial & Engineering Chemistry, 37 (9), 1945, p.865 à 908), le séchage de l'acide acétique introduit en excès à l'étape d'estérification est réalisé avec une variante de la distillation azéotropique hétérogène bien connue de l'homme du métier, dans laquelle une colonne à distiller permet d'éliminer la MEK et les autres composés organiques qui s'accumulent dans l'entraineur.

L'estérification du butanediol par l'acide acétique est mise en œuvre dans une colonne de distillation réactive. Le diester est soutiré en fond de colonne tandis que l'acide acétique en excès et l'eau produite par la réaction d'estérification sont récupérés dans le distillat.

Une première colonne de distillation est alimentée par ce distillat essentiellement composé d'acide acétique, d'eau, mais aussi de MEK et d'autres composés organiques. Le résidu de cette première colonne est constitué d'acide acétique sec et pur, ce dernier étant renvoyé à l'étape d'estérification du 2,3-butanediol. Le distillat de cette première colonne à distiller, un mélange constitué d'entraineur (dans ce cas de l'acétate d'isopropyle), d'acide acétique, d'eau et de MEK et d'autres composés organiques est soutiré et envoyé dans un décanteur.

Ce décanteur permet de séparer une phase aqueuse contenant majoritairement de l'eau, et une phase organique contenant majoritairement l'entraineur.

La phase aqueuse issue du décanteur est envoyée en tant que reflux d'une seconde colonne à distiller. En fond de cette seconde colonne, de l'eau pure, c'est à dire sans entraineur est obtenue et éliminée du procédé. En tête de cette seconde colonne à distiller, l'azéotrope hétérogène composée d'eau et d'entraineur est récupéré, et renvoyé vers le décanteur.

La phase organique issue du décanteur alimente en tant que reflux une troisième colonne à distiller. Cette troisième colonne permet de produire en tête la MEK et des composés organiques qui sont éliminés du procédé, et en fond un mélange d'entraineur, d'eau et d'acide acétique qui est renvoyé à titre de reflux dans la première colonne à distiller.

Cette solution, pour être opérable, suppose que la troisième colonne à distiller soit extrêmement efficace et maintienne la quantité de MEK et des autres composés organiques dans le système à un niveau tel qu'il ne perturbe pas les équilibres liquide-liquide dans le décanteur. Or, les équilibres liquide-vapeur entre la MEK et l'acétate d'isopropyle sont tels qu'il est difficile d'envisager une bonne séparation de la MEK et de l'entraineur, même avec un grand nombre de plateaux théoriques de distillation. D'autres entraineurs plus facilement séparables de la MEK, comme par exemple l'acétate de butyle, ont été envisagés. Néanmoins, ce dernier ne permet pas de séparer efficacement l'eau et l'acide acétique. En conclusion, il est difficile de trouver un entraineur qui permette à la fois une bonne séparation de l'eau et de l'acide acétique, et qui soit facilement séparable de la MEK.

La présente invention permet de remédier au problème d'accumulation des sous-produits de déshydratation du butanediol dans l'acide carboxylique, dans un procédé de production de butadiène à partir de butanediol par estérification puis pyrolyse. De plus, la présente invention est aussi applicable pour un procédé de production de butadiène à partir des différents isomères du butanediol. En effet, la demanderesse a découvert qu'un enchainement particulier de colonnes de distillation pouvait être mis en œuvre pour éliminer de manière efficace l'eau produite à l'étape d'estérification et les composés organiques pouvant s'accumuler dans l'acide carboxylique.

La demanderesse a découvert que son invention pour sécher l'acide carboxylique et éliminer les composés organiques était particulièrement robuste, et pouvait être mis en œuvre même si le liquide pyrolytique était recyclé sans purification préalable à l'étape d'estérification. De plus, la demanderesse a découvert que le fait d'utiliser le liquide pyrolytique sans purification préalable à l'étape d'estérification selon son invention permettait de ne pas accumuler dans l'acide carboxylique, les intermédiaires de pyrolyse et les sous-produits formés à l'étape de pyrolyse.

Ainsi, la demanderesse a découvert qu'un enchainement particulier de colonnes de distillation, par une gestion judicieuse des séparations des sous-produits et de l'ajout de l'entraineur utilisé dans les distillations azéotropiques, différent de celui du procédé de l'art antérieur, pouvait être mis en œuvre pour :
- sécher l'acide carboxylique et le recycler à l'étape d'estérification
- éliminer de manière efficace l'eau
- éliminer de manière efficace les sous-produits de déshydratation quelque soit le butanediol utilisé dans la charge (2,3-butanediol, 1,4-butanediol et/ou 1,3-butanediol)
- éliminer les intermédiaires de pyrolyse et les sous-produits de pyrolyse du diester de 2,3-butanediol, du diester de 1,4-butanediol et du diester de 1,3-butanediol.

L'invention permet donc de simplifier le procédé selon l'art antérieur et réduire ainsi les coûts d'opération et les coûts d'investissement, tout en gardant une flexibilité sur la nature de la charge butanediol alimentant le procédé (1,4-butanediol, 2,3-butanediol, 1,3-butanediol et leurs mélanges).

### OBJET ET INTÉRÊT DE L'INVENTION

L'invention concerne un procédé de production de 1,3-butadiène à partir d'une charge butanediol comprenant au moins :
a) une étape d'estérification du butanediol par un acide carboxylique pour former le diester correspondant, alimentée par ladite charge butanediol et par une alimentation acide carboxylique comprenant une charge acide carboxylique et l'effluent de pyrolyse liquide issu de l'étape b), et produisant au moins un effluent acide carboxylique et un effluent diester, mise en œuvre en distillation réactive en présence d'un catalyseur acide homogène ou hétérogène, opérée à une pression comprise entre 0,01 et 1 MPa ;
b) une étape de pyrolyse de l'effluent diester issu de l'étape a) comprenant une section de réaction et une section de séparation et produisant au moins un effluent de pyrolyse liquide comprenant au moins 50% poids d'acide carboxylique et un effluent de pyrolyse vapeur comprenant plus de 90% poids de butadiène, ladite section de réaction étant opérée à une température comprise entre 500 et 650°C, l'effluent de ladite section de réaction étant refroidi à une température inférieure à 100°C avant d'alimenter ladite section de séparation ;
c) une étape de distillation alimentée par au moins l'effluent acide carboxylique issu de l'étape a), opérée dans une colonne à distiller à une pression au plus égale à 1 MPa, avec une température de tête de colonne comprise entre 0 et 110°C et une température de fond de colonne comprise entre 100 et 120°C, et produisant un distillat aqueux et un résidu acide carboxylique ;
d) une étape de séchage de l'acide carboxylique alimenté au moins par le résidu acide carboxylique issu de l'étape c) et produisant un effluent eau et un produit acide carboxylique alimentant l'étape a).

Un avantage de l'invention est la possibilité de traiter tous les isomères du butanediol (1,4-butanediol, 2,3-butanediol, 1,3-butanediol et leurs mélanges), pour produire du 1,3-butadiène avec un rendement élevé, supérieur à 70%.

Un autre avantage de l'invention est la capacité d'éliminer les différents sous-produits de déshydratation des butanediols, intermédiaires de pyrolyse et sous-produits de pyrolyse avec un nombre d'équipements réduit, réduisant ainsi les coûts d'opération, réduisant les coûts d'investissement et augmentant la flexibilité du procédé vis-à-vis de la charge.

### DESCRIPTION DÉTAILLÉE DE L'INVENTION

### Charge

Conformément à l'invention, le procédé est alimenté par une charge butanediol comprenant au moins du butanediol, avantageusement au moins 90% poids de butanediol. Ledit butanediol est un isomère du butanediol choisi dans la liste constituée par le 1,4-butanediol, le 2,3-butanediol, le 1,3-butanediol et leurs mélanges. Ladite charge butanediol peut également comprendre de l'eau. Ladite charge butanediol peut provenir d'un procédé de fermentation de sucres ou de gaz de synthèse. Ladite charge butanediol peut provenir d'un procédé de synthèse du butanediol à partir d'acétylène et de formaldéhyde.

De préférence pour les besoins de l'invention, l'acide carboxylique est l'acide acétique.

### Étape a) d'estérification du butanediol

Conformément à l'invention, le procédé de production de 1,3-butadiène comprend une étape a) d'estérification du butanediol par un acide carboxylique pour former le diester correspondant alimentée au moins par ladite charge butanediol et par une charge acide carboxylique et produisant au moins un effluent acide carboxylique et un effluent diester, mise en œuvre en distillation réactive en présence d'un catalyseur acide homogène ou hétérogène, de préférence hétérogène.

Cette étape d'estérification peut être réalisée par toute mise en œuvre en distillation réactive bien connue de l'homme du métier.

La charge butanediol est introduite dans la partie supérieure de la distillation réactive, et l'alimentation acide carboxylique comprenant la charge acide carboxylique, l'effluent de pyrolyse liquide issu de l'étape b), avantageusement également le produit acide carboxylique issu de l'étape d), est introduite dans la partie inférieure de la distillation réactive.

Ladite distillation réactive est opérée à une pression comprise entre 0,01 et 1 MPa, de manière préférée à une pression légèrement supérieure à 0,1 MPa. La température de la distillation réactive est comprise entre la température d'ébullition de l'eau produite en tête, et celle du diester produit en fond. Dans le cas où l'acide carboxylique utilisé est l'acide acétique, la température entre la tête et le fond de la distillation réactive varie typiquement entre 100 et 230°C.

La distillation réactive produit en tête un distillat constituant l'effluent acide carboxylique. Ledit effluent acide carboxylique comprend principalement l'eau produite par la réaction d'estérification, l'acide carboxylique introduit en excès et les sous-produits de déshydratation générés. Ledit effluent acide carboxylique peut aussi comprendre des impuretés organiques en provenance de l'effluent de pyrolyse liquide issu de l'étape b) alimenté en mélange avec la charge acide carboxylique.

La distillation réactive produit en fond un résidu constituant l'effluent diester et comprenant principalement le diester de butanediol produit.

Ladite étape a) d'estérification est opérée de telle sorte que la conversion du butanediol en diester de butanediol est supérieure à 95% mol., de préférence supérieure à 99% mol. Ces performances sont atteintes en ajustant les paramètres de fonctionnement de la distillation réactive, tels que les taux de reflux et de rebouillage, et le ratio charge butanediol/charge acide carboxylique de ladite étape a), comme connu de l'Homme de l'art.

Les débits de charge butanediol et d'alimentation acide carboxylique sont ajustés de telle sorte que le ratio molaire acide carboxylique / diol en entrée de l'étape d'estérification est compris entre 2 et 6, de préférence entre 2 et 4, de manière très préférée entre 2 et 3,5.

Le taux de reflux molaire (égal au débit molaire de reflux du condenseur vers la tête de colonne divisé par le débit molaire de distillat) est compris entre 0,5 et 10, de manière préférée entre 0,5 et 4, de manière très préférée entre 1 et 2. Conformément à l'invention, le taux de rebouillage molaire (égal au débit molaire de reflux du rebouilleur vers le fond de colonne divisé par le débit molaire de résidu) est compris entre 0,5 et 10, de manière préférée entre 4 et 10, de manière très préférée entre 5 et 6. Dans un arrangement préféré, ladite distillation réactive comprend une zone mixte de réaction / séparation située entre deux zones de séparation.

Dans cet arrangement, ladite charge butanediol est introduite dans ladite colonne de distillation réactive à un étage intermédiaire, de préférence entre la zone mixte et la zone de séparation située au-dessus de la zone mixte. Ladite alimentation acide carboxylique est introduite dans ladite colonne de distillation réactive à un ou plusieurs étages intermédiaires situés en-dessous de l'étage d'injection de la charge butanediol. De manière préférée, ladite charge acide carboxylique est introduite dans la colonne de distillation réactive à un seul étage intermédiaire, situé entre la zone mixte et la zone de séparation située en-dessous.

Par étage intermédiaire, on entend un étage de la colonne de distillation réactive qui n'est ni le rebouilleur, ni le condenseur. Par au-dessus ou supérieur, on entend en direction du condenseur. Par en-dessous ou inférieur, on entend en direction du rebouilleur.

Chacune desdites zones de séparation comprend des internes connus de l'Homme du métier tels que des plateaux, du garnissage vrac ou structuré, ou une association de ces types d'internes, lesdits internes ou ladite association présentant au global une efficacité de séparation pour chacune desdites zones de séparation d'au moins deux étages théoriques, de préférence comprise entre deux et dix étages théoriques, et de manière préférée comprise entre deux et quatre étages théoriques, de manière à garantir un minimum de rendement et de pureté du diester de diol produit.

De préférence, ladite zone mixte comprend un catalyseur acide hétérogène. Dans un premier arrangement particulier, ladite zone mixte est constituée de plateaux et de sections catalytiques, lesquelles sont situées à l'extérieure de la colonne à distiller, chaque section catalytique étant reliée aux plateaux de ladite zone mixte par le biais d'un soutirage liquide sur un plateau de ladite zone mixte, avec réinjection au plateau inférieur après passage dans ladite section catalytique. Ladite zone mixte comprend avantageusement au plus 20, de préférence au plus 15 sections catalytiques.

Dans un second arrangement particulier, ladite zone mixte est constituée d'internes maintenant ledit catalyseur. Ledit catalyseur est alors maintenu dans ladite zone mixte par les moyens connus de l'homme du métier. De manière non limitative, le catalyseur hétérogène peut être maintenu entre les plaques d'un garnissage structuré, être emprisonné dans des grilles métalliques déposées sur les plateaux de distillation, être emprisonné dans une toile mise en forme de manière à servir de garnissage et établir le transfert entre la phase gaz et la phase liquide, ou bien encore dans un dispositif de distribution particulière des phases liquide et vapeur tel que décrit dans le brevet FR 2,737,131. De manière préférée, ladite zone mixte met en œuvre le dispositif de distribution particulière des phases liquide et vapeur tel que décrit dans le brevet FR 2,737,131. Ce dispositif est préféré car il génère une plus faible perte de charge au sein de la colonne, la phase gaz étant court-circuitée de la zone catalytique. Ce dispositif permet donc de maintenir une plus faible pression en fond de colonne, et donc une plus faible température. Lorsqu'un dispositif de distribution particulière des phases liquide et vapeur tel que décrit dans le brevet FR 2,737,131 est utilisé pour maintenir le catalyseur hétérogène dans la colonne, la zone mixte est constituée d'une alternance de sections de réaction et de sections de séparation. De manière avantageuse, ladite zone mixte comprend, selon ce mode de réalisation, au plus 20, de préférence au plus 15 sections de réaction.

Le temps de séjour de la phase liquide dans chaque section catalytique selon le premier arrangement particulier, ou dans chaque section de réaction dans le second arrangement particulier est avantageusement compris entre 5 et 30 minutes, de manière préférée entre 15 et 25 minutes. De plus, la vitesse superficielle de la phase liquide au sein du lit fixe de catalyseur est avantageusement comprise entre 0,05 et 0,5 cm/s et de manière préférée entre 0,1 et 0,3 cm/s.

Indépendamment du mode de réalisation, le catalyseur acide hétérogène est choisi parmi une résine acide échangeuse d'ions (de type Amberlyst, Amberlite, Dowex, et en particulier une Amberlyst 35, une Amberlyst 36 ou une Amberlyst 70), un oxyde mixte (ZrO2, SnO) ou une zéolithe acide (H-MOR, H-MFI, H-FAU et H-BEA). De manière préférée, ledit catalyseur acide hétérogène est stable à une température supérieure à 130°C, de manière préférée supérieure à 150°C, de manière très préférée supérieure à 170°C.

Les catalyseurs acides utilisés pour catalyser la réaction d'estérification activent également les réactions de déshydratation, notamment aux températures d'opération de ladite étape a), produisant de la MEK ou du THF ou du 2-butèn-1-ol suivant l'isomère de butanediol présent dans la charge butanediol.

Le temps de séjour dans ladite colonne de distillation réactive, défini comme le volume de la distillation réactive divisé par le débit volumique de ladite charge diol et de ladite charge acide carboxylique, est avantageusement compris entre 0,5 h et 10 h, de préférence entre 0,5 h et 5 h, et de manière préférée entre 1 h et 2 h.

De manière préférée, la MMH (Mole par Mole par Heure, correspondant au débit molaire de diol dans la charge diol divisé par le nombre de moles de catalyseur présent au sein de ladite zone mixte) est comprise entre 0,05 et 25 h-1, préférentiellement entre 0,15 et 20 h-1.

### Étape b) de pyrolyse du diester de butanediol

Conformément à l'invention, le procédé de production de 1,3-butadiène comprend une étape b) de pyrolyse de l'effluent diester issu de l'étape a) comprenant une section de réaction et une section de séparation et produisant au moins un effluent de pyrolyse liquide comprenant au moins 50%poids d'acide carboxylique et un effluent de pyrolyse vapeur comprenant plus de 90% poids de butadiène, ladite section de réaction étant opérée à une température comprise entre 500 et 650°C, l'effluent de ladite section de réaction étant refroidi à une température inférieure à 100°C avant d'alimenter ladite section de séparation.

La réaction de pyrolyse transforme une mole de diester de butanediol en une mole de 1,3-butadiène et libère ainsi deux moles d'acide carboxylique. Plus de 70%mol du diester de butanediol est converti en 1,3-butadiène. Préférentiellement, plus de 80%mol du diester de butanediol est converti en 1,3-butadiène. Ledit réacteur de pyrolyse est opéré à une température comprise entre 500 et 650°C, de préférence entre 550 et 600°C, de manière préférée entre 575 et 585°C. Le temps de contact optimal au sein dudit réacteur est fonction de la pression partielle du diester de butanediol injecté dans ledit réacteur. Il est typiquement de 1 seconde pour une pression partielle de diester de diol de 0,1 MPa, et de 7 secondes pour une pression partielle de diester de diol de 0,04 MPa.

Ladite étape b) de pyrolyse selon l'invention comprenant également au moins une section de séparation alimentée par ledit effluent de pyrolyse, refroidi à une température inférieure à 100°C, de manière à produire au moins un effluent de pyrolyse liquide et un effluent de pyrolyse vapeur qui peuvent être aisément séparées au sein d'un ballon séparateur gaz-liquide. L'effluent de pyrolyse issu dudit réacteur de pyrolyse est refroidi rapidement à une température inférieure à 100°C, de préférence inférieure à 50°C, de manière à limiter la formation de produits de dégradation par exemple par réaction de Diels-Alder de 1,3-butadiène sur lui-même pour former du VinylCycloHexène (VCH).

Ledit effluent de pyrolyse vapeur comprend plus de 90% poids, de préférence plus de 95% poids de 1,3-butadiène (sans considérer l'éventuel diluant inerte utilisé pour abaisser la pression partielle de diester de butanediol au sein du réacteur de pyrolyse). Ledit effluent de pyrolyse vapeur peut également contenir des composés organiques légers, issus de la pyrolyse de l'acide carboxylique, comme par exemple dans le cas où l'acide carboxylique est de l'acide acétique, du méthane, du monoxyde de carbone, du dioxyde de carbone, du cétène, de l'hydrogène ou encore de l'éthane. Ledit effluent de pyrolyse vapeur peut être comprimé et/ou refroidi de manière à condenser le 1,3-butadiène. Les composés organiques non condensables issus de la pyrolyse de l'acide carboxylique sont ainsi éliminés en tête d'un séparateur gaz-liquide sous forme d'un effluent de composés légers. Le 1,3-butadiène peut ensuite subir une ou plusieurs étapes de purification ultimes bien connues de l'homme du métier. On peut citer de manière non limitative la purification sur tamis ou sur une argile, ou encore un lavage à l'eau. Ceci permet d'éliminer les dernières traces d'impuretés et d'obtenir un effluent 1,3-butadiène, lequel comprend plus de 99%, de manière préférée plus de 99,5% de 1,3-butadiène, qui est le produit du procédé.

Ledit effluent de pyrolyse liquide est composé majoritairement d'acide carboxylique. Par majoritairement, on entend au moins 50% poids, et de préférence au moins 70% poids. Il peut également comprendre d'autres composés organiques, comme par exemple du diester de butanediol non converti, des intermédiaires de pyrolyse (c'est-à-dire les molécules de diester de butanediol ayant perdu un fragment acide carboxylique sur les deux nécessaires à la formation du 1,3-butadiène), et d'éventuels sous-produits de pyrolyse. La nature de ces sous-produits dépend bien entendu de la nature de la charge introduite dans le réacteur de pyrolyse.

De nombreux intermédiaires de pyrolyse et impuretés sont produits. A titre d'illustration, dans le cas où de l'acide acétique est utilisé pour réaliser l'estérification du 2,3-butanediol à l'étape a), l'effluent de pyrolyse liquide comprend du diacétate de 2,3-butanediol (1,5%pds.), des intermédiaires de pyrolyse tels que, le méthylvinylcarbinol acétate (MVCA 0,8%pds.), le méthyléthylcétoneénol acétate (MEKEA 2,4%pds.) et le crotylacétate (CA 3,3%pds.) et des sous-produits tels que le VCH (2,2%pds), la MEK (1,4%pds.) ou la méthylacétylacétone (MAA 0.9%pds.). L'effluent de pyrolyse liquide contient donc de nombreux intermédiaires de pyrolyse ayant une température d'ébullition proche de celle de l'acide acétique, et formant des azéotropes homogènes (maximum et minimum) avec ce dernier. La purification de l'acide acétique avant son recyclage à l'étape d'estérification ne peut donc se faire par simple distillation.

Dans un premier mode de l'invention, l'effluent de pyrolyse liquide est purifié avant d'être recyclé vers l'étape a) d'estérification en mélange avec la charge acide carboxylique. Cette purification permet d'éliminer les intermédiaires et sous-produits de pyrolyse formés dans l'étape b).

L'effluent de pyrolyse liquide est purifié par tout mode bien connu de l'homme du métier. Il peut par exemple être purifié par distillation azéotropique hétérogène, en utilisant l'eau comme entraineur, tel que décrit dans le document « Pilot-plant conversion of 2,3-butylene glycol diacetate to 1,3-butadiene » dans le journal « Industrial and Engineering Chemistry » volume 37, N°9.

Dans un second mode de réalisation de l'invention, l'effluent de pyrolyse liquide est directement recyclé vers l'étape a) d'estérification en mélange avec la charge acide carboxylique, sans purification. Le diester de butanediol non converti à l'issue de l'étape b) de pyrolyse est ainsi récupéré en fond de ladite colonne de distillation réactive et renvoyé vers l'alimentation de ladite étape b) de pyrolyse, améliorant le rendement global en 1,3-butadiène du procédé.

Dans ce second mode de réalisation de l'invention, les intermédiaires de pyrolyse et certains sous-produits de pyrolyse qui sont difficilement séparables de l'acide carboxylique sont convertis totalement ou partiellement dans les conditions opératoires de l'étape a) d'estérification en d'autres produits plus facilement séparables de l'acide carboxylique et qui s'évacuent en tête de ladite colonne de distillation réactive vers l'étape c) de distillation. Par exemple, dans le cas où l'acide carboxylique utilisé est l'acide acétique pour estérifier le 2,3-butanediol, la méthylacétylacétone (MAA) et la méthylacétylacétone (MEKEA) formées dans l'étape b) de pyrolyse sont hydrolysés dans les conditions de l'étape a) d'estérification en acide acétique et en MEK, qui s'évacuent en tête de ladite colonne de distillation réactive vers l'étape c) de distillation.

### Étape c) de distillation

Conformément à l'invention, l'étape c) de distillation est alimentée par l'effluent acide carboxylique issu de l'étape a). L'étape c) de distillation produit un distillat aqueux comprenant les sous-produits de déshydratation et de pyrolyse et ne comprenant pas plus de 10% poids d'acide carboxylique qui est éliminé du procédé et un résidu acide carboxylique qui est séché à l'étape d).

L'alimentation de l'étape c) comprend de l'eau produite par la réaction d'estérification, de l'acide carboxylique et des composés organiques issus de la déshydratation de la charge butanediol, tels que par exemple le THF ou la MEK ou le 2-butèn-1-ol. Ladite alimentation peut également comprendre des intermédiaires de pyrolyse, des sous-produits de pyrolyse formés dans l'étape b) de pyrolyse, recyclés et non convertis dans l'étape a) d'estérification, ainsi que des composés organiques qui sont issus de la conversion des intermédiaires de pyrolyse et des sous-produits de pyrolyse dans l'étape a) d'estérification.

Ladite étape c) de distillation est opérée dans une colonne à distiller à une pression au plus égale à 1 MPa, et de préférence à une pression comprise entre 0,1 et 0,2 MPa. La température de la tête de colonne est comprise entre 0 et 110°C, de préférence comprise entre 50°C et 100°C. Dans ces conditions, le distillat aqueux produit en tête de ladite colonne à distiller est composé majoritairement d'eau et des sous-produits organiques. Par majoritairement, on entend que la teneur en acide carboxylique ne dépasse pas 10% poids, de préférence ne dépasse pas 5% poids. La température de fond de colonne est comprise entre 100 et 120°C, de préférence entre 100°C et 115°C. Ainsi, le résidu acide carboxylique produit en fond de cette première colonne est appauvri en sous-produits organiques et constitué principalement d'eau et d'acide carboxylique.

De manière surprenante, l'agencement de l'étape a) d'estérification et de l'étape b) de pyrolyse avec recyclage de l'effluent de pyrolyse liquide issu de l'étape b) vers ladite étape a) conduit, du fait des différentes réactions ayant lieu dans l'étape a) et l'étape b), à la production d'un effluent acide carboxylique issu de l'étape a) pouvant être séparé d'une part en un distillat aqueux comportant de l'eau et les sous-produits de déshydratation et de pyrolyse et d'autre part un résidu acide carboxylique constitué d'eau et d'acide carboxylique.

Le résidu acide carboxylique produit en fond de cette colonne est exempt de composés organiques et constitué d'eau et d'acide acétique. Il constitue la charge de l'étape d). Ce mode de fonctionnement de la colonne permet d'éliminer plus de 98% des sous-produits organiques et de limiter les pertes en acide acétique entre 0 et10% en masse, de préférence entre 0 et 5%, et de manière très préférée entre 0 et 1% en masse.

En fonction de l'isomère ou des isomères de butanediol présent dans ladite charge butanediol, une démixtion peut se produire dans le condenseur de la colonne à distiller mise en œuvre dans l'étape c) de distillation. Dans le cas où une démixtion se produit, l'Homme du métier peut augmenter le taux de reflux afin de se situer en dehors de la zone de démixtion et aboutir de nouveau à un mélange homogène. Cependant, dans un arrangement avantageux où une démixtion se produit, la phase aqueuse est renvoyée en reflux vers la colonne et la phase riche en composés organiques est soutirée comme distillat.

### Étape d) de séchage de l'acide carboxylique

Conformément à l'invention, une étape d) de séchage de l'acide carboxylique est alimentée par le résidu acide carboxylique issu de l'étape c) de distillation et produit un effluent eau et un produit acide carboxylique.

Ladite étape d) de séchage est une séparation eau/acide carboxylique et peut être mise en œuvre par exemple par distillation azéotropique hétérogène en présence d'un entraineur.

L'entraineur qui forme un hétéroazéotrope avec le mélange acide carboxylique-eau est de préférence choisi dans le groupe constitué par les alcanes, les composés aromatiques, les cétones, les esters, et les mélanges de ces derniers. De préférence, l'entraineur est choisi dans le groupe constitué par le n-dodécane, le mesitylène, la 3-pentanone, la 2-hexanone, la 4-méthyl-2-pentanone, la 2-pentanone, la cyclopentanone, la cyclohexanone, la diisobutylcétone, l'isopropyle acétate, le n-propyle acétate, l'éthyle acétate, le n-butyle acétate, l'iso-butyle acétate et les mélanges de ces derniers.

Selon un arrangement préférentiel, l'étape d) comprend en sortie de la distillation azéotropique, une étape de décantation du mélange eau-acide carboxylique-entraineur, permettant de séparer la phase aqueuse (eau) de la phase organique comprenant l'entraineur et l'acide carboxylique ; la phase organique étant renvoyée dans la colonne de distillation azéotropique. Cet arrangement préférentiel est particulièrement adapté dans le cas où l'entraineur est très peu miscible à l'eau. De préférence, en particulier lorsque l'acide carboxylique est l'acide acétique, l'entraineur est l'isopropyle acétate.

Selon un autre arrangement préférentiel, quelques soient l'acide carboxylique et l'entraineur, on peut utiliser une colonne de distillation en sortie du décanteur, pour séparer l'entraineur resté dans la phase aqueuse lors de la décantation, cet entraineur étant renvoyé dans le décanteur.

Le produit acide carboxylique est recyclé vers l'étape a) d'estérification en mélange avec la charge acide carboxylique.

### EXEMPLES

### Exemple 1 [comparatif] Séchage de l'acide acétique par distillation azéotropique hétérogène et élimination de la MEK

*Cet exemple montre qu'un arrangement selon l'art antérieur, dans lequel un entraineur est ajouté à l'effluent acide carboxylique issu de l'étape d'estérification, cet entraineur pouvant être facilement séparé de l'acide carboxylique, pose des problèmes au niveau de la séparation MEK*/*entraineur.*

Un effluent acide carboxylique issu d'une étape d'estérification conduite dans une distillation réactive alimente une colonne de distillation azéotropique hétérogène. L'entraineur utilisé est l'isopropyl acétate. Cet effluent acide carboxylique contient de la MEK, de l'acide acétique et de l'eau. L'entraineur est ajouté à la charge.

La colonne de distillation azéotropique hétérogène est opérée à pression atmosphérique, avec 30 étages théoriques un taux de rebouillage massique de 7,57. L'alimentation est effectuée dans le tiers supérieur de la colonne.

La phase organique du condenseur de cette colonne est soutirée et contient 96,2% de la MEK alimentée dans ladite colonne ainsi que l'entraineur. Cette phase organique alimente une seconde colonne destinée à séparer la MEK de l'entraineur.

Cette séparation est très difficile car les courbes de rosée et de bulle du mélange MEK/isopropyl-acétate sont très proches et un très grand nombre d'étages (plus de 50 étages théoriques) sont nécessaires.

**Tableau 1 : Bilan matière à la colonne de distillation azéotropique hétérogène de séchage de l'acide acétique selon art antérieur**

| | **Alimentation** | **Résidu** | **Condensat phase organique** | **Condensat phase aqueuse** |
|---|---|---|---|---|
| **Température (°C)** | 94.7 | 115.1 | 112.4 | 112.4 |
| **Débit massique** | | | | |
| **MEK** | 0.106 | 0.00 | 0.102 | 3.73E-03 |
| **Acide acétique** | 1.00 | 0.599 | 0.334 | 0.066 |
| **Eau** | 0.52 | 7.24E-03 | 0.209 | 0.307 |
| **Isopropyl acétate** | 1.81 | 0.00 | 1.80 | 5.74E-04 |

### Exemple 2 [conforme] recyclage de l'effluent de pyrolyse liquide en estérification

*Cet exemple montre que l'enchainement des étapes d'estérification et de pyrolyse, avec recyclage de l'effluent de pyrolyse liquide conduit à la transformation de certains sous-produits de pyrolyse en produits facilement séparables (MEK) dans les étapes de séparation de l'effluent acide carboxylique selon l'invention. Ces sous-produits auraient sinon nécessité un traitement dédié. Il y a ainsi synergie entre l'enchainement estérification*/*pyrolyse et le recyclage de l'effluent liquide de pyrolyse et l'enchainement d'étapes de séparations alimenté par l'effluent acide carboxylique selon l'invention.*

Cet exemple montre la possibilité de recycler l'effluent de pyrolyse liquide selon l'invention.

Une charge butanediol constituée de 2,3-butanediol alimente une étape d'estification. L'effluent diester comprend le diacétate de 2,3-butanediol formé.

L'effluent diester alimente une étape de pyrolyse, laquelle comprend un four de pyrolyse opéré à 580°C avec un temps de contact d'environ 2 s. L'effluent de pyrolyse est rapidement refroidi à 45°C et se condense en un effluent de pyrolyse liquide. La partie non condensée, qui constitue l'effluent de pyrolyse vapeur, comprend 97,5% poids de 1,3-butadiène. La composition de l'effluent de pyrolyse liquide est indiquée dans le tableau 2.

**Tableau 2 : Composition massique et molaire de l'effluent de pyrolyse liquide. 2,3-BDODiAc=diacétate de 2,3-butanediol, BDE=butadiène, VCH=vinylclohexène, MEK=methyl ethyl ketone, MAA=méthylacétylacétone, MVCA=méthyl vinyl carbinol acétate, MEKEA=méthyl éthyl cétone énol acétate, CA=crotyl acétate.**

| | **% massique** | **% molaire** |
|---|---|---|
| **AA** | 79,60% | 83,54% |
| **2,3-BDOdiAc** | 2,81% | 1,02% |
| **BDE** | 9,01% | 10,51% |
| **VCH** | 0,62% | 0,36% |
| **MEK** | 0,57% | 0,50% |
| **MVCA** | 0,95% | 0,52% |
| **MEKEA** | 3,49% | 1,93% |
| **CA** | 2,64% | 1,46% |
| **MAA** | 0,31% | 0,17% |

Deux tests d'estérification du 2,3-butanediol par l'acide acétique ont été réalisés. Un test a été réalisé avec de l'acide acétique pur et l'autre de l'effluent de pyrolyse liquide décrit ci-dessus. Ces tests ont été conduits dans un réacteur batch d'un volume de 30 mL à pression atmosphérique, équipé d'un condenseur. La température est constante et régulée à 110°C grâce à un fluide caloporteur dans une double enveloppe. Les réactions sont opérées en présence d'un catalyseur Amberlyst 36, avec un nombre de fonction acide H+ présent à une concentration de 2,2% molaire par rapport au 2,3-butanediol. Ces réactions ont été effectuées avec un ratio molaire acide acétique/2,3-butanediol de 6. Ces tests ont permis de suivre la cinétique de réaction d'estérification, mais aussi l'évolution des différentes impuretés et intermédiaires de pyrolyse au cours du temps.

On constate aussi par les résultats présentés dans le tableau ci-dessous que les impuretés présentes dans l'effluent de pyrolyse liquide, certaines ont évolué au cours du temps.

**Tableau 3: Résultats des deux tests d'estérification de 2,3-BDO avec de l'acide acétique pur et avec un effluent de pyrolyse liquide**

| | **Acide acétique** | **Liquide de pyrolyse** |
|---|---|---|
| | C^{initiale} (mol.L⁻¹) | C^{finale} (mol.L⁻¹) |
| BDE | 1,2551 | 1,2305 |
| VCH | 0,0864 | 0,0542 |
| MEK | 0,0794 | 0,6371 |
| MVCA | 0,1323 | 0,1407 |
| MEKEA | 0,4862 | 0,0117 |
| CA | 0,3678 | 0,2905 |
| MAA | 0,0432 | 0,0000 |

Les concentrations de VCH et MVCA ont peu évolué. Le CA n'évolue également pas significativement. Par contre, on montre ici que la MEKEA et la MAA disparaissent quasi totalement dans les conditions de l'estérification pour donner de la MEK. En effet, la disparition de la MEKEA et MAA correspond à 0,5177 mol.L-1 et la formation de MEK correspond à 0,5577 mol.L-1, ce qui est dans l'erreur de mesure.

On démontre ainsi que certains intermédiaires de pyrolyse et certains sous-produits de pyrolyse sont partiellement ou totalement convertis en d'autres produits dans les conditions de l'estérification, qui sont plus facilement séparables de l'acide acétique, ce qui permet d'éviter leur accumulation. Ainsi, l'enchainement des étapes estérification/pyrolyse avec recyclage de l'effluent de pyrolyse liquide selon l'invention permet, par la transformation induite de certains sous-produits, d'améliorer le fonctionnement des étapes de séparation réalisées sur l'effluent acide carboxylique.

### Exemple 3 [conforme] Elimination de la MEK, sous-produits de pyrolyse avant séchage de l'acide acétique par distillation azéotropique hétérogène

*Cet exemple montre qu'en l'absence d'un entraineur, les sous-produits compris dans l'effluent acide carboxylique peuvent être séparés par distillation en produisant un résidu acide carboxylique ne comprenant quasiment que de l'acide carboxylique et de l'eau, lequel résidu pourra être traité par distillation azéotropique hétérogène sans difficulté.*

Le tableau ci-dessous, montre le bilan matière d'une colonne de distillation, opérée à pression atmosphérique, avec 15 étages théoriques d'équilibre un taux de reflux massique de 1,48 et un taux de rebouillage massique de 0,47 où l'alimentation est faite à l'étage 5.

**Tableau 4 : Bilan matière à la colonne de distillation azéotropique d'élimination de la MEK (étape c)), avant séchage de l'acide acétique par distillation azéotropique hétérogène (étape d)).**

| | **Alimentation** | **Distillat** | **Résidu** |
|---|---|---|---|
| Température (°C) | 94.7 | 83.4 | 99.6 |
| Débit massique | | | |
| MEK | 0.106 | 0.105 | 0.001 |
| Acide Acétique | 1.000 | 0.030 | 0.970 |
| MVCA | 0.081 | 0.081 | 0.000 |
| CA | 0.108 | 0.078 | 0.030 |
| Eau | 0.523 | 0.091 | 0.432 |
| VCH | 1.69E-05 | 1.69E-05 | 0.00E+00 |

La MEK est éliminée à 99%, le MVCA à 100%, le CA à 72%, le VCH à 100%, l'eau à 17%. Cette simulation révèle une perte en acide acétique de 3% qui est tout à fait acceptable.

Le résidu exempt de MEK peut être distillé de manière efficace par distillation azéotropique hétérogène.

### Exemple 4 [conforme] Elimination de la MEK, sous-produits de pyrolyse avant séchage de l'acide acétique par distillation azéotropique hétérogène

*Cet exemple montre qu'en l'absence d'un entraineur, les sous-produits compris dans l'effluent acide carboxylique peuvent être séparés par distillation en produisant un résidu acide carboxylique ne comprenant quasiment que de l'acide carboxylique et de l'eau, lequel résidu pourra être traité par distillation azéotropique hétérogène sans difficulté. Dans cet exemple, la colonne est opérée de telle manière qu'une démixtion a lieu dans le condenseur.*

Le tableau ci-dessous, montre le bilan matière d'une colonne de distillation, opérée entre 1 et 2 bar, avec 25 étages théoriques d'équilibre un taux de reflux massique de 2,15 et un taux de rebouillage massique de 0,9 où l'alimentation est faite à l'étage 13, sachant que le premier étage est en tête de colonne.

Les vapeurs de tête de la colonne sont condensées jusqu'à 86°C et envoyées vers un ballon de reflux dans lequel deux phases liquides coexistent. La phase organique est soutirée comme distillat et est exempt d'acide acétique. La phase aqueuse (de composition Eau 96 % wt, MEK 1,6 % wt, MVCA 1,1 % wt) est renvoyée en reflux vers la colonne de distillation.

**Tableau 5 : Bilan matière à la colonne de distillation azéotropique d'élimination de la MEK, avant séchage de l'acide acétique.**

| | **Alimentation** | **Distillat** | **Résidu** |
|---|---|---|---|
| Température (°C) | 96.6 | 86 | 114.5 |
| Débit massique | | | |
| MEK | 0.02955 | 0.02955 | 0.00000 |
| MEKEA | 0.04677 | 0.04677 | 0.00000 |
| Acide Acétique | 1.00000 | 0.00003 | 0.99997 |
| MVCA | 0.15535 | 0.15535 | 0.00000 |
| CA | 0.08946 | 0.08946 | 0.00000 |
| Eau | 0.57785 | 0.01618 | 0.56167 |
| VCH | 0.00003 | 0.00003 | 0.00000 |

La MEK est éliminée à 100%, le MVCA à 100%, le CA à 100%, le VCH à 100%, l'eau à 28%. Cette simulation révèle une perte en acide acétique de 0.003% qui est tout à fait négligeable.

Le résidu exempt de MEK, CA, MVCA peut être distillé de manière efficace par distillation azéotropique hétérogène et la totalité de l'acide acétique peut être ainsi récupérée afin d'être recyclée et valorisé.

### Exemple 5 [conforme] Elimination de THF, sous-produits de pyrolyse avant séchage de l'acide acétique par distillation azéotropique hétérogène

*Cet exemple montre qu'en l'absence d'un entraineur, les sous-produits compris dans l'effluent acide carboxylique peuvent être séparés par distillation en produisant un résidu acide carboxylique ne comprenant quasiment que de l'acide carboxylique et de l'eau, lequel résidu pourra être traité par distillation azéotropique hétérogène sans difficulté.*

Cet exemple montre la simulation du procédé d'élimination du THF et des sous-produits de pyrolyse avant le séchage de l'acide acétique par distillation azéotropique hétérogène, selon l'invention.

Le tableau ci-dessous, montre le bilan matière d'une colonne de distillation, opérée à pression atmosphérique, avec 15 étages théoriques d'équilibre un taux de reflux massique de 10 et un taux de rebouillé massique de 0.42 où l'alimentation est faite à l'étage 5.

**Tableau 6 : Bilan matière à la colonne de distillation azéotropique d'élimination du THF et le sous-produit de pyrolyse, avant séchage de l'acide acétique.**

| | **Alimentation** | **Distillat** | **Résidu** |
|---|---|---|---|
| **Température (°C)** | 94.7 | 83.4 | 99.6 |
| **Débit massique** | | | |
| **Acide Acétique** | 1.0000 | 0.0185 | 0.9815 |
| **MonoAc(= du 1,4)** | 0.0869 | 0.0869 | 0.0000 |
| **WATER** | 1.2293 | 0.0374 | 1.1919 |
| **THF** | 1.21 E-04 | 1.21 E-04 | 1.61E-10 |
| **VCH** | 0.0024 | 0.0024 | 0.0000 |

Le THF est éliminé à 100%, le mono acétylé issue de la pyrolyse est éliminé à 100%, le est aussi éliminé VCH à 100%, l'eau à 3%. Cette simulation révèle une perte en acide acétique de 2% qui est tout à fait acceptable.

Le résidu est majoritairement composé d'eau et d'acide acétique. Ainsi, ce résidu peut être distillé de manière efficace par distillation azéotropique hétérogène en présence d'un entraineur.

## Revendications

1. Procédé de production de 1,3-butadiène à partir d'une charge butanediol comprenant au moins :
a) une étape d'estérification du butanediol par un acide carboxylique pour former le diester correspondant, alimentée par ladite charge butanediol et par une alimentation acide carboxylique comprenant une charge acide carboxylique et l'effluent de pyrolyse liquide issu de l'étape b), et produisant au moins un effluent acide carboxylique et un effluent diester, mise en œuvre en distillation réactive en présence d'un catalyseur acide homogène ou hétérogène, opérée à une pression comprise entre 0,01 et 1 MPa ;
b) une étape de pyrolyse de l'effluent diester issu de l'étape a) comprenant une section de réaction et une section de séparation et produisant au moins un effluent de pyrolyse liquide comprenant au moins 50% poids d'acide carboxylique et un effluent de pyrolyse vapeur comprenant plus de 90% poids de butadiène, ladite section de réaction étant opérée à une température comprise entre 500 et 650°C, l'effluent de ladite section de réaction étant refroidi à une température inférieure à 100°C avant d'alimenter ladite section de séparation ;
c) une étape de distillation alimentée par au moins l'effluent acide carboxylique issu de l'étape a), opérée dans une colonne à distiller à une pression au plus égale à 1 MPa, avec une température de tête de colonne comprise entre 0 et 110°C et une température de fond de colonne comprise entre 100 et 120°C, et produisant un distillat aqueux comprenant les sous-produits de l'étape a) et de l'étape b) d'une part, et un résidu acide carboxylique d'autre part ;
d) une étape de séchage de l'acide carboxylique alimenté au moins par le résidu acide carboxylique issu de l'étape c) et produisant un effluent eau et un produit acide carboxylique alimentant l'étape a).

2. Procédé selon la revendication 1 dans lequel ladite charge butanediol comprend au moins 90% poids de butanediol choisi dans la liste constituée par le 1,4-butanediol, le 1,3-butanediol et le 2,3-butanediol pris seul ou en mélange.

3. Procédé selon l'une des revendications précédentes dans lequel ledit acide carobxylique est l'acide acétique.

4. Procédé selon l'une des revendications précédentes dans lequel la distillation réactive de l'étape a) comprend une zone mixte de réaction / séparation située entre deux zones de séparation.

5. Procédé selon la revendication précédente dans lequel ladite zone mixte comprend un catalyseur acide hétérogène choisi parmi une résine acide échangeuse d'ions, un oxyde mixte ou une zéolithe acide.

6. Procédé selon l'une des revendications précédentes dans lequel le temps de séjour dans ladite colonne de distillation réactive de l'étape a), défini comme le volume de la distillation réactive divisé par le débit volumique de ladite charge diol et de ladite charge acide carboxylique, est compris entre 0,5 h et 10 h.

7. Procédé selon l'une des revendications 4 à 6 dans lequel la MMH, correspondant au débit molaire de diol dans la charge diol divisé par le nombre de moles de catalyseur présent au sein de ladite zone mixte, est comprise entre 0,05 et 25 h-1.

8. Procédé selon l'une des revendications précédentes dans lequel ledit effluent de pyrolyse liquide de l'étape b) est purifié avant l'être recyclé vers l'étape a) en mélange avec la charge acide carboxylique.

9. Procédé selon l'une des revendications précédentes dans lequel ledit distillat aqueux de ladite étape c) ne comprend pas plus de 10% poids d'acide carboxylique.

10. Procédé selon l'une des revendications précédentes dans lequel l'étape c) est opérée dans une colonne à distiller dont la température de la tête de colonne est comprise entre 50°C et 100°C, et la température de fond de colonne est comprise entre 100°C et 115°C.

11. Procédé selon l'une des revendications précédentes dans lequel ladite étape d) est mise en œuvre par distillation azéotropique hétérogène en présence d'un entraineur.

12. Procédé selon la revendication précédente dans lequel ledit entraineur de l'étape d) est l'isopropyle acétate.

## Patentansprüche

1. Verfahren zur Herstellung von 1,3-Butadien aus einer Butandiol-Charge, umfassend mindestens:
a) einen Schritt der Veresterung des Butandiols durch eine Carbonsäure, um den entsprechenden Diester zu bilden, der mit der Butandiol-Charge und mit einer Carbonsäure-Versorgung versorgt wird, umfassend eine Carbonsäure-Charge und den flüssigen Pyrolyseabfluss aus dem Schritt b), wobei mindestens ein Carbonsäure-Abfluss und ein Diester-Abfluss erzeugt wird, der in reaktiver Destillation im Beisein eines homogenen oder heterogenen sauren Katalysators eingesetzt und bei einem Druck zwischen 0,01 und 1 MPa durchgeführt wird;
b) einen Schritt der Pyrolyse des Diester-Abflusses aus Schritt a), umfassend einen Reaktionsabschnitt und einen Trennungsabschnitt, wobei mindestens ein flüssiger Pyrolyse-Abfluss, umfassend mindestens 50 Gew.-% Carbonsäure, und ein dampfförmiger Pyrolyse-Abfluss, umfassend mehr als 90 Gew.-% Butadien, erzeugt wird, wobei der Reaktionsabschnitt bei einer Temperatur zwischen 500 und 650 °C betrieben wird, wobei der Abfluss des Reaktionsabschnitts auf eine Temperatur unter 100 °C gekühlt wird, bevor der Trennungsabschnitt versorgt wird;
c) einen Destillationsschritt, der mit mindestens einem Carbonsäure-Abfluss aus Schritt a) versorgt wird, der in einer Destillationskolonne bei einem Druck höchstens gleich 1 MPa mit einer Kolonnen-Kopftemperatur zwischen 0 und 110 °C und einer Kolonnen-Sumpftemperatur zwischen 100 und 120 °C durchgeführt wird, und ein wässriges Destillat erzeugt, umfassend die Unterprodukte des Schritts a) und des Schritts b) einerseits und einen Carbonsäurerest andererseits;
d) einen Trocknungsschritt der Carbonsäure, der zumindest mit dem Carbonsäurerest aus Schritt c) versorgt wird und einen Wasserabfluss und ein Carbonsäureprodukt, das den Schritt a) versorgt, erzeugt.

2. Verfahren nach Anspruch 1, bei dem die Butandiol-Charge mindestens 90 Gew.-% Butandiol umfasst, gewählt aus der Liste, gebildet durch 1,4-Butandiol, 1,3-Butandiaol und 2,3-Butandiol allein oder im Gemisch.

3. Verfahren nach einem der vorhergehenden Ansprüche, bei dem die Carbonsäure Essigsäure ist.

4. Verfahren nach einem der vorhergehenden Ansprüche, bei dem die reaktive Destillation des Schritts a) eine Mischzone Reaktion/Trennung zwischen zwei Trennungszonen umfasst.

5. Verfahren nach dem vorhergehenden Anspruch, bei dem die Mischzone einen heterogenen sauren Katalysator umfasst, der unter einem sauren Ionenaustauschharz, einem Mischoxid oder einem sauren Zeolith ausgewählt ist.

6. Verfahren nach einem der vorhergehenden Ansprüche, bei dem die Aufenthaltszeit in der reaktiven Destillationskolonne des Schritts a), die als das Volumen der reaktiven Destillation dividiert durch die volumenbezogenen Menge der Diol-Charge und der Carbonsäure-Charge definiert ist, zwischen 0,5 h und 10 h beträgt.

7. Verfahren nach einem der Ansprüche 4 bis 6, bei dem die MMH entsprechend der Molmenge an Diol in der Diol-Charge dividiert durch die Katalysator-Molzahl, die in der Mischzone vorhanden ist, zwischen 0,05 und 25 h-1 beträgt.

8. Verfahren nach einem der vorhergehenden Ansprüche, bei dem der flüssige Pyrolyseabfluss des Schritts b) gereinigt wird, bevor er zu Schritt a) im Gemisch mit der Carbonsäure-Charge rückgeführt wird.

9. Verfahren nach einem der vorhergehenden Ansprüche, bei dem das wässrige Destillat des Schritts c) nicht mehr als 10 Gew.-% Carbonsäure umfasst.

10. Verfahren nach einem der vorhergehenden Ansprüche, bei dem der Schritt c) in einer Destillationskolonne durchgeführt wird, deren Kolonnen-Kopftemperatur zwischen 50 °C und 100 °C und Kolonnen-Bodentemperatur zwischen 100 °C und 115 °C beträgt.

11. Verfahren nach einem der vorhergehenden Ansprüche, bei dem der Schritt d) durch heterogene azeotrope Destillation im Beisein eines Schleppmittels eingesetzt wird.

12. Verfahren nach dem vorhergehenden Anspruch, bei dem der Trainer des Schritts d) Isopropylazetat ist.

## Claims

1. Method for producing 1,3-butadiene from a butanediol feedstock that comprises at least:
a) A step for esterification of butanediol by a carboxylic acid to form the corresponding diester, fed by said butanediol feedstock and by a carboxylic acid feed comprising a carboxylic acid feedstock and the liquid pyrolysis effluent obtained from step b), and producing at least a carboxylic acid effluent and a diester effluent, implemented in reactive distillation in the presence of a homogeneous or heterogeneous acid catalyst, operated at a pressure of between 0.01 and 1 MPa;
b) A step for pyrolysis of the diester effluent obtained from step a) comprising a reaction section and a separation section and producing at least a liquid pyrolysis effluent that comprises at least 50% by weight of carboxylic acid and a vapor pyrolysis effluent that comprises more than 90% by weight of butadiene, with said reaction section being operated at a temperature of between 500 and 650°C, the effluent of said reaction section being cooled to a temperature that is less than 100°C before feeding said separation section;
c) A step for distillation fed by at least the carboxylic acid effluent obtained from step a), operated in a distillation column at a pressure that is at most equal to 1 MPa, with a temperature at the top of the column of between 0 and 110°C and a temperature at the bottom of the column of between 100 and 120°C, and producing an aqueous distillate comprising the by-products of step a) and step b), on the one hand, and a carboxylic acid residue, on the other hand;
d) A step for drying the carboxylic acid that is fed at least by the carboxylic acid residue obtained from step c) and producing a water effluent and a carboxylic acid product that feeds step a).

2. Method according to Claim 1, wherein said butanediol feedstock comprises at least 90% by weight of butanediol selected from the list that consists of 1,4-butanediol, 1,3-butanediol and 2,3-butanediol, taken by themselves or in a mixture.

3. Method according to one of the preceding claims, wherein said carboxylic acid is acetic acid.

4. Method according to one of the preceding claims, wherein the reactive distillation of step a) comprises a reaction/separation mixed zone located between two separation zones.

5. Method according to the preceding claim, wherein said mixed zone comprises a heterogeneous acid catalyst that is selected from among an ion-exchange acid resin, a mixed oxide, or an acid zeolite.

6. Method according to one of the preceding claims, wherein the dwell time in said reactive distillation column of step a), defined as the volume of the reactive distillation divided by the volumetric flow rate of said diol feedstock and said carboxylic acid feedstock, is between 0.5 h and 10 h.

7. Method according to one of Claims 4 to 6, in which the MMH, corresponding to the diol molar flow rate in the diol feedstock divided by the number of moles of catalyst present within said mixed zone, is between 0.05 and 25 h-1.

8. Method according to one of the preceding claims, wherein said liquid pyrolysis effluent of step b) is purified before being recycled to step a) in a mixture with the carboxylic acid feedstock.

9. Method according to one of the preceding claims, wherein said aqueous distillate of said step c) does not comprise more than 10% by weight of carboxylic acid.

10. Method according to one of the preceding claims, wherein step c) is operated in a distillation column whose temperature of the top of the column is between 50°C and 100°C, and the temperature of the bottom of the column is between 100°C and 115°C.

11. Method according to one of the preceding claims, wherein said step d) is implemented by heterogeneous azeotropic distillation in the presence of a driver.

12. Method according to the preceding claim, wherein said driver of step d) is isopropyl acetate.
